# EUROPEAN PATENT APPLICATION

(11) **EP 3 978 603 A1**
(43) Date of publication of application: **06.04.2022**
(21) Application number: 21179731.1
(22) Date of filing: 16.06.2021
(51) Int. Cl.: C12N 9/10

(54) **A BUTYRIC ACID-PRODUCING BACILLUS SUBTILIS STRAIN, METHOD OF CONSTRUCTION AND APPLICATION THEREOF**

(30) Priority: 30.09.2020 CN 202011065916
(71) Applicant: Tianjin University, Tianjin 300072 (CN)
(72) Inventor: BAI, Liang, Nankai District, 300072 (CN); CHENG, Xiaoming, Nankai District, 300072 (CN); HUANG, He, Nankai District, 300072 (CN); KANG, Guangbo, Nankai District, 300072 (CN)
(74) Representative: Murgitroyd & Company

(57) **Abstract**

The present disclosure describes a butyric acid-producing *Bacillus subtilis* strain, a method of construction and application thereof. Autolysis-related genes and acetic acid metabolism-related genes are deleted on the genome of the chassis cell *B. Subtilis.* Meanwhile, the genome further contains a butyric acid synthesis-related gene *BCoAT.* In the present disclosure, autolysis-related genes and acetic acid metabolism-related genes in a chassis *B. subtilis* strain are knocked out, the butyric acid synthesis-related gene *BCoAT* is inserted, the butyric acid metabolic pathway of the *B. subtilis* is optimized, a bypass gene is knocked out and a heterogenous metabolic pathway is introduced. Finally, the butyric acid yield of the optimized *B. subtilis* strain increases by 6.95 times compared with that of the original strain.

SEQUENCE LISTING

<110>  Tianjin University

<120> A Butyric Acid-producing Bacillus subtilis Strain, Method of
Construction and Application Thereof

<130>  1

<160>  2

<170>  PatentIn version 3.5

<210>  1
<211>  84
<212>  DNA
<213>  Artificial Sequence

<220>
<223>  sgRNA targeting site

<400>  1
gttttagagc tagaaatagc aagttaaaat aaggctagtc cgttatcaac ttgaaaaagt      60

ggcaccgagt cggtgctttt tttg                                              84


<210>  2
<211>  1347
<212>  DNA
<213>  Faecalibacterium prausnitzii

<400>  2
atggatttta cggaattgta tgcgcagaag aaaatgacag ctgaccaggc tgccgcgttg      60

gtcaaaagcg gcgactgggt ggattacggc tgggctgtga acacccccgt tgcagtggac     120

gctgccattg caaagcgtct gcccgagctg gaggatgtca acttccgcgg cggcatcctg     180

atgtgggtgc ccgccatctt ccagatcgag gatcccgctg cccacatgac ctggaacagc     240

tggcacatgg gcggcattga gcgcaaggcc attgcccagg gcttctcctt ctactccccc     300

atccgctact ctgaactgcc ccgctactat cgcgacagct ctgaccccgt ggatgtggcg     360

gtgttccagg tgacccccat ggacgagcac ggctatttta actttggccc ctgtgcttcc     420

cacctgggtg ccgtctgcga taaggccaaa aagatcatcg ttgaggtcaa tcggaacatg     480

cccaaatgcc tgggcggcac cgagaactgg gtgcacatct cccaggttgc cggtgttgtg     540

gagggcagca acccgcccat cggccagatg gccgctgccg gtgccgccac cgaggtcgat     600

ctgaaggtgg caaacctcat cgtcccgcag atccccgacg gtgcctgcct gcagctgggc     660

atcggcggta tgcccaacgc catcggcaac ctgatcgcgc agagcgacct gaaggatctg     720

ggcgtgcaca ccgagatgta tgtggatgcc tttgtggata ttgcaaaggc aggcaagatc     780

accggccgcc acaagaatct ggacaagggc cgtcaggtct atgcctttgg tgccggcacc     840

cagaagatgt atgattacct gaacgacaac ccggagtgca tggccgctcc cgtggagtac     900

accaacgaca tccgcagcat ctccgccatc gacaacttca tctccatcaa caatgcggtg 960

gacattgacc tgttcggtca ggtgaacgcg gagagcgcag gcatcaagca catttccggt 1020

gcaggtggtc agctggactt tgttctgggc gcataccttt ccaacggcgg caagagcttc 1080

atctgcctgt cctctacctt tatgaataaa aagaccggca agctggagag ccgcatccgt 1140

cccaccctgg aaaacggcag catcgtcacc gacacccgcg caaacgtgca ctacctgtgc 1200

accgagtacg gctgtgtgaa cctgaaggga ctgaccagct gggaaaaggc agaagcactg 1260

atcagcgtgg cacaccccga tttccgggac gagctgatcg ccgaagccga aaaactgcac 1320

atctggcgca ggagcaacaa gcgctga 1347

## Description

### CLAIM OF PRIORITY

This application claims the benefit of CN Application No. CN202011065916.7, filed on September 30, 2020. The entire contents of the foregoing are incorporated herein by reference.

### TECHNICAL FIELD

The present disclosure relates to the field of microbial fermentation, and particularly relates to a butyric acid-producing *B. subtilis* strain, method of construction and application thereof.

### BACKGROUND

Butyric acid (BA) is one of short-chain fatty acids (SCFAs) generated by intestinal flora metabolism and mainly exists in a free form. Mass butyric acid-producing microorganisms inhabit human and animal gastrointestinal tracts. Studies have confirmed that butyric acid has many critical physiological functions, i.e., participating in material metabolism, promoting intestinal structure development, and enhancing immunity and other physiological activities.

At present, butyric acid, as an organic acid widely applied to industries of food, chemistry, medicines, etc., is mainly produced by chemical synthesis. Meanwhile, with the enhancement of people's environmental awareness and the improvement of petroleum energy values, butyric acid biosynthesis is attracting more and more attention. However, a problem of butyric acid biosynthesis is end product inhibition. Microbiological fermentation leads to a lower yield of butyric acid and poor economic benefit, so it is impossible to replace chemical synthesis. Therefore, in order to further improve the yield of butyric acid fermentation, it is necessary to improve the fermentation method, fermentation media, fermentation strain, etc.

CN109355318A discloses a method for producing butyric acid through fermentation. The method comprises the following steps: activating *Clostridium tyrobutyricum* to prepare a seed solution; preparing a liquid medium by using a mixture of mannitol and glucose as a carbon source, and sterilizing; and inoculating the liquid medium with the seed solution and performing cell fermentation at a temperature of 35-39 °C, a pH of 5-7 and a revolution speed of 100-300 rpm to obtain butyric acid. The method provided by the present disclosure may improve the conversion rate and selectivity of the target product butyric acid, thereby reducing the cost of subsequent separation and purification. However, the method requires a large amount of glucose and mannitol as a carbon source for fermentation. If waste is used as a carbon source, there are a large amount of toxicity inhibitors, so the fermentation method has a high cost.

CN108441462A discloses *B. subtilis* ΔWB800 and a preparation method thereof. By knocking out autolysis-related genes *skfA, sdpC, lytC* and *xpf* of the *B. subtilis* ΔWB800, an autolysis inhibiting strain ΔWB800 is obtained. The strain has excellent performance, e.g., heat resistance, acid resistance and bile salt tolerance, and has good industrial application prospects. Although the original *B. subtilis* has a certain capacity of producing butyric acid, the yield of butyric acid is relatively low. Therefore, the present disclosure only improves heat resistance, acid resistance and other performance of the strain, but not to remarkably improve the butyric acid yield of the *B. subtilis.*

Accordingly, it is of great significance in the art to provide a high butyric acid-producing *B. subtilis* strain.

### SUMMARY

In view of the problems existing in the prior art, the present disclosure describes a butyric acid-producing *Bacillus subtilis* (*B. subtilis)* strain, method of construction and application thereof. This strain has high butyric acid yield, mature construction method and high success rate, which is suitable for fermentation and production of butyric acid. For this purpose, the present disclosure describes the technical solution below:
In the first aspect, the present disclosure describes a butyric acid-producing B. *subtilis* strain. Autolysis-related genes and acetic acid metabolism-related genes are deleted on the genome of the strain; and meanwhile, the genome further contains a butyric acid synthesis-related gene *BCoAT.*

Wherein, the butyric acid synthesis-related gene *BCoAT,* i.e., gene butyryl -CoA: acetate CoA-transferase (*BCoAT*), is a gene involved in butyric acid synthesis non-classical pathway. In the present disclosure, by knocking out butyric acid autolysis-related genes and bypass metabolism-related genes and at the same time inserting the butyric acid synthesis-related gene *BCoAT,* the butyric acid yield of the *B. subtilis* strain can be remarkably improved.

As a preferred technical solution of the present disclosure, the genome further includes the thiolase (THL) gene, the β-hydroxybutyryl-CoA dehydrogenase (BHBD) gene, the butyryl-CoA dehydrogenase (BCD) gene and the crotonase (CRO) gene.

Preferably, the thiolase gene, the β-hydroxybutyryl-CoA dehydrogenase gene, the butyryl-CoA dehydrogenase gene, the crotonase gene and the gene *BCoAT* on the genome are sequentially connected.

It is indicated by analysis on genomes of butyric acid synthesis microorganisms that an arrangement mode of related enzyme genes in a central pathway of butyric acid synthesis is diversified and associated with the distribution of enzyme genes in a final pathway. Most butyryl phosphate transferases and butyrate kinase genes in a traditional BK pathway exist in pairs and are arranged adjacent to the enzyme genes in the central pathway.

In a butyric acid-producing bacterium having encoding gene *BCoAT,* distribution of the enzyme genes in the final pathway is irregular, and the distribution and arrangement of the enzyme genes in the central pathway are diversified, which may affect the synthesis of BA. In the present disclosure, the thiolase gene, the β-hydroxybutyryl-CoA dehydrogenase gene, the butyryl-CoA dehydrogenase gene, the crotonase gene and the gene *BCoAT* are sequentially connected, and the butyric acid yield of the resultant *B. subtilis* strain is the highest.

Preferably, the autolysis-related genes include any one or a combination of at least two of *skfA, sdpC, lytC* or *xpf,* preferably a combination of *skfA* and *sdpC.*

Preferably, the acetic acid metabolism-related genes include gene *acdA* and/or gene *ackA.*

Acetic acid (AA) is also a product of the metabolism of *B. subtilis* and shares a same substrate acetyl-CoA with butyric acid, which may weaken the metabolic flux in the synthesis direction of butyric acid. Therefore, blocking AA synthesis pathway and reducing AA synthesis is an effective way to promote butyric acid synthesis. Since ACDA gene encodes acetyl coenzyme A ligase and regulates acetyl coenzyme A to produce acetic acid, the production of AA can be blocked by knocking out the gene.

In the second aspect, the present disclosure describes a method for constructing the *B. subtilis* strain described in the first aspect. The method comprises the following steps:
using two-plasmids CRISPR/Cas9 gene editing system to perform gene editing on a chassis cell, knocking out autolysis-related genes and acetic acid metabolism-related genes, and inserting a butyric acid synthesis-related gene *BCoAT* to obtain the *B. subtilis* strain.

The two-plasmids CRISPR/Cas9 gene editing systems mainly comprises a pCas plasmid and a pTargetF plasmid, wherein the pCas plasmid generates Cas protein, the pTargetF plasmid generates guide RNA, and the two types of plasmids are combined to guide the Cas protein to specifically edit a genome. The present disclosure mainly uses the pTargetF plasmid as a skeleton to construct a targeting and guiding plasmid. Such plasmid mainly comprises a promoter, sgRNA and homologous arm repair segments. Existing spectinomycin is selected as a selection marker gene, and transcription of the sgRNA is completed by a constitutive P43 promoter.

As a preferred technical solution of the present disclosure, the genome of the chassis cell includes thiolase gene, β-hydroxybutyryl-CoA dehydrogenase gene, butyryl-CoA dehydrogenase gene and crotonase gene, which are sequentially connected. In the present disclosure, the chassis cell that is used is *B. subtilis* SCK6.

Preferably, in the two-plasmids CRISPR/Cas9 gene editing systems, plasmids used comprise a pCas plasmid and a pTargetF plasmid.

Preferably, the selection marker on the pCas plasmid includes a kanamycin marker gene.

Preferably, the pCas plasmid further includes a temperature-sensitive replicon region *rep*101. The pCas plasmid contains the temperature-sensitive replicon region rep101. When the culture temperature exceeded 30 °C, the host cell containing the plasmid was sensitive to temperature. Therefore, when cultured overnight at 37 °C, the host cell in which the pCas plasmid is eliminated might be selected out.

For knocking out the autolysis-related genes and the acetic acid metabolism-related genes, the pTargetF plasmid comprises a promoter, a selection marker and sgRNA.

Preferably, the promoter includes a P43 promoter.

Preferably, the selection marker includes a spectinomycin marker gene.

Preferably, gene sequence of the sgRNA is shown as SEQ ID NO.1, specifically: 
GTTTTAGAGCTAGAAATAGCAAGTTAAAATAAGGCTAGTCCGTTATCAACTT
GAAAAAGTGGCACCGAGTCGGTGCTTTTTTTG;
 and

For inserting the butyric acid synthesis-related gene *BCoAT,* the pTargetF plasmid further includes homologous arms and a butyric acid synthesis-related gene *BCoAT.*

Preferably, a nucleotide sequence of the butyric acid synthesis-related gene *BCoAT* is shown as SEQ ID NO.2.

The SEQ ID NO.2 is:

ATGGATTTACGGAATTGTATGCGCAGAAGAAAATGACAGCTGACCAG

GCTGCCGCGTTGGTCAAAAGCGGCGACTGGGTGGATTACGGCTGGGCTGT
GAACACCCCCGTTGCAGTGGACGCTGCCATTGCAAAGCGTCTGCCCGAGC
TGGAGGATGTCAACTTCCGCGGCGGCATCCTGATGTGGGTGCCCGCCATCT
TCCAGATCGAGGATCCCGCTGCCCACATGACCTGGAACAGCTGGCACATGG
GCGGCATTGAGCGCAAGGCCATTGCCCAGGGCTTCTCCTTCTACTCCCCCA
TCCGCTACTCTGAACTGCCCCGCTACTATCGCGACAGCTCTGACCCCGTGG
ATGTGGCGGTGTTCCAGGTGACCCCCATGGACGAGCACGGCTATTTTAACT
TTGGCCCCTGTGCTTCCCACCTGGGTGCCGTCTGCGATAAGGCCAAAAAGA
TCATCGTTGAGGTCAATCGGAACATGCCCAAATGCCTGGGCGGCACCGAGA
ACTGGGTGCACATCTCCCAGGTTGCCGGTGTTGTGGAGGGCAGCAACCCG
CCCATCGGCCAGATGGCCGCTGCCGGTGCCGCCACCGAGGTCGATCTGAA
GGTGGCAAACCTCATCGTCCCGCAGATCCCCGACGGTGCCTGCCTGCAGCT
GGGCATCGGCGGTATGCCCAACGCCATCGGCAACCTGATCGCGCAGAGCG
ACCTGAAGGATCTGGGCGTGCACACCGAGATGTATGTGGATGCCTTTGTGG
ATATTGCAAAGGCAGGCAAGATCACCGGCCGCCACAAGAATCTGGACAAG
GGCCGTCAGGTCTATGCCTTTGGTGCCGGCACCCAGAAGATGTATGATTAC
CTGAACGACAACCCGGAGTGCATGGCCGCTCCCGTGGAGTACACCAACGA
CATCCGCAGCATCTCCGCCATCGACAACTTCATCTCCATCAACAATGCGGTG
GACATTGACCTGTTCGGTCAGGTGAACGCGGAGAGCGCAGGCATCAAGCA
CATTTCCGGTGCAGGTGGTCAGCTGGACTTTGTTCTGGGCGCATACCTTTCC
AACGGCGGCAAGAGCTTCATCTGCCTGTCCTCTACCTTTATGAATAAAAAG
ACCGGCAAGCTGGAGAGCCGCATCCGTCCCACCCTGGAAAACGGCAGCAT
CGTCACCGACACCCGCGCAAACGTGCACTACCTGTGCACCGAGTACGGCT
GTGTGAACCTGAAGGGACTGACCAGCTGGGAAAAGGCAGAAGCACTGAT
CAGCGTGGCACACCCCGATTTCCGGGACGAGCTGATCGCCGAAGCCGAAA
AACTGCACATCTGGCGCAGGAGCAACAAGCGCTGA.

Exemplarily, the genome of the *B. subtilis* in the present disclosure may be edited by the following method:
1. extracting the pCas plasmid and transforming the pCas plasmid into a *B. subtilis* strain, and selecting by using a kanamycin-containing resistant LB plate to obtain a positive single colony;
2. preparing competent cells from the positive single colony, transforming the competent cells into pTargetF plasmids, coating a double resistance (i.e., kanamycin and spectinomycin)-containing LB plate with transformation liquid, conducting inverted overnight culture at a constant temperature of 30 °C, and finally selecting to obtain recombinant *B. subtilis* containing the pCas plasmid and the pTargetF plasmid at the same time;
3. inoculating a liquid LB medium containing arabinose of which the final concentration is 10-15 mM (e.g., 11 mM, 12 mM, 13 mM, or 14 mM) with the recombinant strain, culturing in a shaking table at 30 °C and 200 rpm for 16-20 h (e.g., 17 h, 18 h or 19 h), and then extracting the genome for PCR and validating by sequencing; and
4. collecting the strain validated by sequencing and conducting plasmid elimination, specifically:
   after validating the success of gene editing, inoculating a culture medium containing kanamycin and IPTG of which concentrations are 0.03-0.06 g/L (e.g., 0.035 g/L, 0.04 g/L, 0.045 g/L or 0.05 g/L) and 0.4-0.6 mM (e.g., 0.42 mM, 0.45 mM, 0.48 mM, 0.5 mM or 0.55 mM) respectively with the strain, culturing in a shaking table at 30 °C and 200 rpm for 8-16 h (e.g., 9 h, 10 h, 12 h or 14 h), diluting and then coating a spectinomycin-containing LB plate, confirming whether a colony is cured, i.e., whether the pTargetF plasmid is eliminated, by determining the sensitivity of the colony to spectinomycin; and
   using the colony of which the pTargetF plasmid is eliminated for a second round of gene editing until the editing work ends.

Wherein, the pCas plasmid contains a temperature sensitive replicon region rep101 °C. When the culture temperature exceeds 30 °C, the pCas plasmid is temperature sensitive. Therefore, the host cell containing the pCas plasmid is grown at 37 °C overnight to ensure that the pCas plasmid is eliminated. The pCas plasmid always exists in the editing process, and it is eliminated after editing. The strain without pCas plasmid is the resultant genetic engineering strain, which can be used for butyric acid synthesis in fermentation.

As a preferred technical solution of the present disclosure, the method of construction includes the following steps:
(1) constructing a two-plasmids CRISPR/Cas9 gene knockout editing system, wherein the gene editing system includes a pCas plasmid containing a kanamycin marker gene and a pTargetF plasmid containing a spectinomycin marker gene;
(2) transforming the gene editing system in step (1) into a chassis cell, knocking out an autolysis-related gene *skfA,* and eliminating the pCas plasmid and the pTargetF plasmid after verifying the success of gene editing, wherein the genome of the chassis cell includes a thiolase gene, a β-hydroxybutyryl-CoA dehydrogenase gene, a butyryl-CoA dehydrogenase gene and a crotonase gene;
(3) respectively knocking out an autolysis-related gene *sdpC,* an acetic acid metabolism-related gene *acdA* and an acetic acid metabolism-related gene *ackA*;
(4) constructing a two-plasmids CRISPR/Cas9 gene editing system for insertion, wherein the gene editing system includes a pCas plasmid and a pTargetF plasmid, and the pTargetF plasmid contains a P43 promoter, a spectinomycin marker gene, sgRNA, homologous arms and a butyric acid synthesis-related gene *BCoAT,* gene sequence of the sgRNA is shown as SEQ ID NO.1, and a nucleotide sequence of the butyric acid synthesis-related gene *BCoAT* is shown as SEQ ID NO.2; and
(5) transforming the gene editing system in step (4) into the chassis cell of which the gene *skfA,* the gene *sdpC,* the gene *acdA* and the gene *ackA* knocked out, and inserting the butyric acid synthesis-related gene *BCoAT* into a downstream of the crotonase gene to obtain the *B. subtilis.*

In the third aspect, the present disclosure further describes application of the B. *subtilis* strain as described in the first aspect in the preparation of butyric acid.

Exemplarily, the present disclosure may perform fermentation on the strain and product determination by using the following method, specifically:

### (1) Fermentation culture of the strain

A fermentation flask is inoculated with the constructed *B. subtilis* strain at an initial inoculum size of 0.5-1.5 % (e.g., 0.6 %, 0.8 %, 1 %, 1.2 %, 1.3 % or 1.4 %); and
culturing and fermenting are performed in a shaking table at 37 °C and 180-220 rpm (e.g., 190 rpm, 200 rpm or 210 rpm) for 20-24 h (e.g., 21 h, 22 h, 22.5 h or 23 h), and the pH value of a fermentation broth is monitored by using a pH electrode in the fermentation process, wherein the pH value at the initial stage of fermentation is about 7, and the final pH value of the fermentation broth is about 4 since acid is continuously generated;

### (2) Determination of short-chain fatty acids (SCFAs)

Pretreatment: after fermentation ends, the content of the SCFAs in the fermentation broth is determined, and a collected supernatant test sample is acidized; and the culture supernatant is taken and put into a centrifuge tube, a sulfuric acid solution with a mass fraction of 50% and diethyl ether are added, followed by mixing in a shaking table at room temperature and 180-220 rpm (e.g., 190 rpm, 200 rpm or 210 rpm) for a certain period, the mixture is centrifuged, the supernatant is taken, and anhydrous calcium chloride (CaCl₂) is added for dehydrating treatment, centrifuging is performed, and the supernatant is taken for gas chromatography-mass spectrometry (GC-MS) analysis.

The numerical range of the invention includes not only the point values listed above, but also any point values between the above numerical ranges not listed. For the sake of length and conciseness, the present disclosure no longer exhaustively lists the specific point values within the range.

Compared with the prior art, the present disclosure at least has the beneficial effects below:
(1) In the present disclosure, the two-plasmids CRISPR/Cas9 gene editing systems are used for gene editing, the autolysis-related genes and the acetic acid metabolism-related genes in the chassis cell *B. subtilis* are knocked out. Meanwhile, the butyric acid synthesis-related gene *BCoAT* is inserted into the genome of the *B. subtilis,* the bypass gene is knocked out, and the butyric acid metabolic pathway of the *B. subtilis* is optimized to obtain the *B. subtilis* strain capable of highly producing butyric acid.
(2) According to the present disclosure, the *B. subtilis* SCK6 is used as an original strain, the autolysis-related genes *skfA* and *sdpC* and the bypass metabolism genes *acdA* and *ackA* are knocked out, the butyric acid synthesis non-classical pathway gene *BCoAT* is introduced and an insertion site of the gene *BCoAT* is optimized, and the butyric acid yield of the optimized engineering *B. subtilis* is up to 1.53 g/L and is increased by 6.95 times compared with that of an original bacterium.

### BRIEF DESCRIPTION OF THE DRAWINGS

Fig. 1 is a structural schematic diagram of a blank pTargetF plasmid in a two-plasmids editing system in Example 1;
Fig. 2 is a structural schematic diagram of a pTargetF plasmid in a two-plasmids editing system during gene insertion in Example 1;
Fig. 3 is a schematic diagram of arrangement sequences of gene clusters strain BsS-5, strain BsS-6 and strain BsS-7 constructed in Examples 1-3;
Fig. 4 is a gel electrophoretogram obtained when it is verified that strain BsS-5, strain BsS-6 and strain BsS-7 constructed in Examples 1-3 contain the gene *BCoAT,* wherein a lane M represents a DNA marker, lanes 1 and 2 represent verification results of strain BsS-5, lanes 4 and 5 represent verification results of strain BsS-6, lanes 7 and 8 represent verification results of strain BsS-7, and lanes 3, 6 and 9 represent blank controls;
Fig. 5 is a bar graph showing butyric acid yields of strain BsS, strain BsS*-ΔskfA,* strain BsS*-ΔsdpC* and strain BsS-1;
Fig. 6 shows curve graphs of butyric acid and acetic acid yields of strain BsS-1, strain BsS-2, strain BsS-3 and strain BsS-4 as a function of time, wherein graph A shows the curves corresponding to strain BsS-1, graph B shows the curves corresponding to strain BsS-2, graph C shows the curves corresponding to strain BsS-3, and graph D shows the curves corresponding to strain BsS-4; and
Fig. 7 is a bar graph showing butyric acid yields of strain BsS-4, strain BsS-5, strain BsS-6 and strain BsS-7.

### DETAILED DESCRIPTION

The technical solution of the present disclosure is further described in conjunction with accompanying drawings by specific embodiments, but the examples described below are only simple examples of the present disclosure and do not represent or limit the protection scope of rights of the present disclosure, and the protection scope of the present disclosure is defined by the claims.

In the following examples, unless otherwise specified, adopted experimental methods are all common technical methods in the art. Unless otherwise specified, adopted reagents, consumables and strains can all be purchased from conventional manufacturers in the art.

In the following examples, a method for editing the genome of a *B. subtilis* strain includes the following steps:
1. a strain containing a pCas plasmid was taken from -80 °C, a fresh liquid LB medium was inoculated with the strain, the strain was cultured in a constant temperature shaking table at 37 °C and 200 rpm for 8-10 h, and then a kanamycin-containing solid LB plate was coated with the cultured strain, then a single colony was picked up to be cultured overnight in a fresh LB medium, and the pCas plasmid was extracted;
2. the pCas plasmid was transformed into the *B. subtilis,* selecting was conducted by using a kanamycin-containing resistant LB plate to obtain a positive single colony, competent cells were prepared, and then a pTargetF plasmid was transformed;
3. a kanamycin and spectinomycin-containing LB plate was coated with transformation liquid, inverted overnight culture was conducted at a constant temperature of 30 °C, and the recombinant *B. subtilis* strain containing the pCas plasmid and the pTargetF plasmid was screened out;
4. a liquid LB medium containing arabinose of which the final concentration was 10 mM was inoculated with the recombinant strain and the recombinant strain was cultured in a shaking table at 30 °C and 200 rpm for 16 h, and a genome was extracted for PCR and sequencing; and
5. after verifying the success of the gene edit, the strain obtained in step 4 was inoculated to a 2 mL culture medium containing 0.05 g/L of kanamycin and 0.5 mM of IPTG, the strain was cultured in a shaking table at 37 °C and 200 rpm for 16 h, the cultured strain was diluted and then an antibiotics-containing LB plate was coated with the diluted strain; and
it was confirmed whether the colony was cured, i.e., whether the pTargetF plasmid was eliminated, by determining the sensitivity of the colony to spectinomycin, and the colony of which the pTargetF plasmid was eliminated was used for a second round of gene editing until the editing work ended.

In the following examples, an adopted fermentation culture method and an adopted product determination method are as follows:
(1) Fermentation culture of engineered strain
   a 0.5 L fermentation flask was inoculated with the engineered strain, wherein a fermentation medium was prepared by adding 0.4 g/L of MgSO₄·7H₂O, 2 g/L of (NH₄)₂SO₄, 1.0 g/L of K₂HPO₄·3H₂O and 0.02 g/L of FeSO₄·7H₂O and supplementing 1-3% of glucose. Inoculation was conducted at an initial inoculum size of 1%, namely a 5 mL seed solution was cultured and fermented in a shaking table at 37 °C and 200 rpm for about 24 h, and a pH value of a fermentation broth was monitored by using a pH electrode in the fermentation process;
(2) Determination of short-chain fatty acids (SCFAs)

### 1. Pretreatment

After fermentation ended, the content of the SCFAs in the fermentation broth was determined, and a collected supernatant test sample was acidized. Specifically:
2ml culture supernatant was put into 5ml PE centrifuge tubes, added with 0.4ml 50% sulfuric acid solution and ether, incubated in a shaking table at room temperature and 200 rpm for 45min, then centrifuged at 3000rpm for 5min. The supernatant was put into another sterile centrifuge tube and dehydrated with anhydrous calcium chloride. The supernatant was taken for gas chromatography-mass spectrometry.

### 2. GC-MS analysis

An adopted chromatographic column was an Agilent 123-7032 DB-WAX quartz capillary column (30 m×320 µm×0.25 µm).

Heating procedures: the initial temperature of the column was 60 °C and kept for 2 min, the column temperature was raised to 220 °C at a rate of 10 °C/min and kept for 20 min, helium gas served as a carrier gas, a flow rate was 1 ml/min, a split ratio was 20:1, an injection volume was 2 µl, and an initial temperature of an injection port was 250 °C.

Mass spectroscopy conditions: an EI ion source had an energy of 70 eV and a temperature of 230 °C, a quadrupole had a temperature of 150 °C, solvent delay time was 2 min, and a mass scan range m/z was 20-150.

### Example 1

This example provides a butyric acid-producing *B. subtilis* strain.

The strain used *B. subtilis* SCK6 (BsS) as an original strain. This original strain was purchased from Wuhan Miaoling Bioscience&Technology Co., Ltd. By using the constructed two-plasmids editing systems, the genes *skfA, sdpC, acdA* and *ackA* were knocked out respectively; and
then by using the two-plasmids editing system, the gene *BCoAT* RS-06650 was inserted in the downstream of gene cluster of crotonase (CRO); and the resultant B. *subtilis* strain was designated as strain BsS-5.

Wherein, the gene *BCoAT* was derived from an intestinal butyric acid-producing bacterium *Faecalibacterium prausnitzii* A2-165 in an NCBI database, and had Gene ID of 34752126 and a specific sequence shown as SEQ ID NO.2.

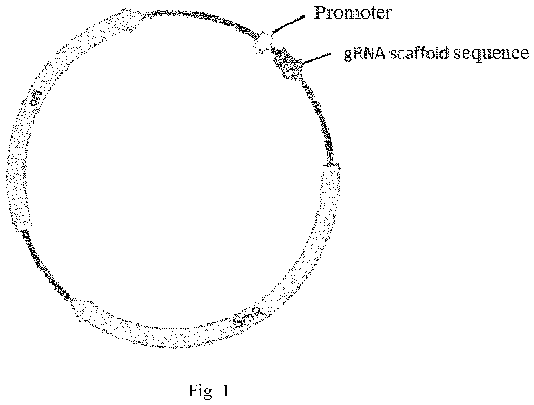

The schematic diagram of the blank pTargetF plasmid in the two-plasmids editing system is shown in Fig. 1.

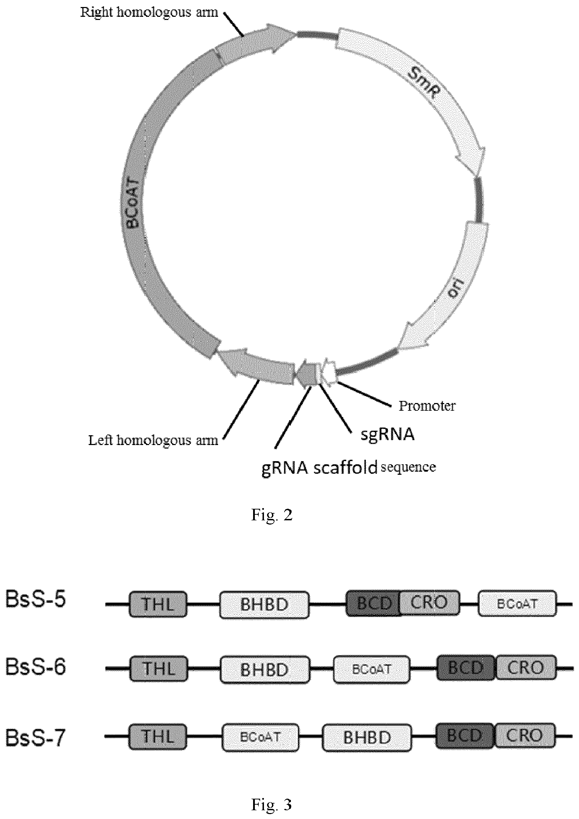

The schematic diagram of the pTargetF plasmid in the two-plasmids editing system during insertion of the gene *BCoAT* is shown in Fig. 2, wherein a sequence of sgRNA is shown as SEQ ID NO.1.

### Example 2

Different from Example 1, the gene *BCoAT* was inserted in the downstream of a gene cluster of β-hydroxybutyryl-CoA dehydrogenase (BHBD), other steps were the same as those of Example 1, and the resultant *B. subtilis* was designated as strain BsS-6.

### Example 3

Different from Example 1, the gene *BCoAT* was inserted in the downstream of a gene cluster of thiolase (THL), other steps were the same as those of Example 1, and the resultant *B. subtilis* was designated as strain BsS-7.

In genomes of the strains obtained in Examples 1-3, the arrangement sequences of the gene clusters for encoding THL, BHBD, BCD, CRO and *BCoAT* are shown in Fig. 3.

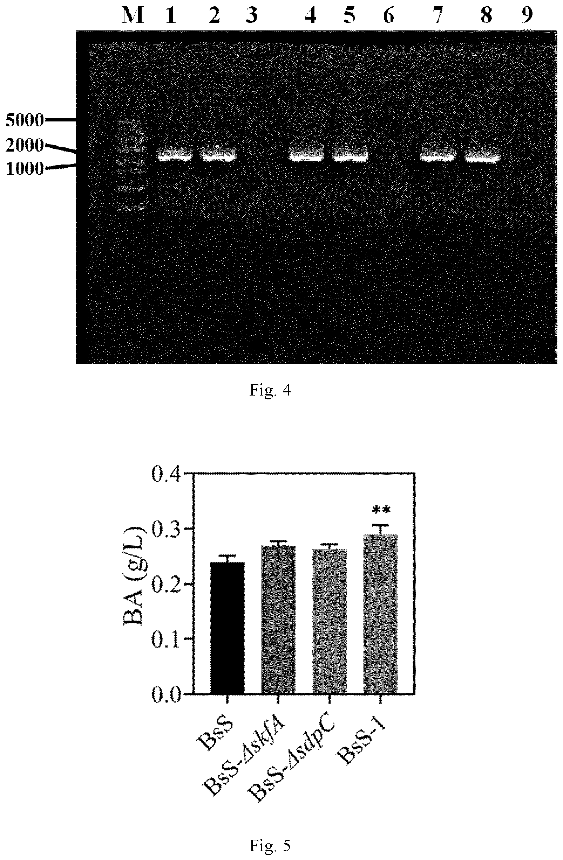

In Examples 1-3, after the gene *BCoAT* was inserted, the genomes were extracted for verification, the obtained gel electrophoresis map is shown in Fig. 4, and it may be known from Fig. 4 that the gene *BCoAT* was successfully inserted in strain BsS-5, strain BsS-6 and strain BsS-7.

### Comparative example 1

Different from Example 1, only gene *skfA* in *B. subtilis* provided by this comparative example was knocked out, which was designated as strain BsS*-ΔskfA.*

### Comparative example 2

Different from Example 1, only gene *sdpC* in *B. subtilis* provided by this comparative example was knocked out, which was designated as strain BsS*-ΔsdpC.*

### Comparative example 3

Different from Example 1, only gene *skfA* and gene *sdpC* in *B. subtilis* provided by this comparative example were knocked out, which was designated as strain BsS-1.

### Comparative example 4

Different from Example 1, gene *skfA,* gene *sdpC* and gene *acdA* in *B. subtilis* provided by this comparative example were knocked out, which was designated as strain BsS-2, i.e., based on strain BsS-1, the gene *acdA* was further knocked out.

### Comparative example 5

Different from Example 1, gene *skfA,* gene *sdpC* and gene *ackA* in *B. subtilis* provided by this comparative example were knocked out, which was designated as strain BsS-3, i.e., based on strain BsS-1, the gene *ackA* was further knocked out, and the *B. subtilis* was designated as strain BsS-3.

### Comparative example 6

Different from Example 1, gene *skfA,* gene *sdpC,* gene *acdA* and gene *ackA* in *B. subtilis* provided by this comparative example were knocked out, which was designated as strain BsS-4, i.e., based on strain BsS-1, the gene *acdA* and the gene *ackA* were further knocked out, and the *B. subtilis* was designated as strain BsS-4.

Information of the strains provided by the above examples and comparative examples is summarized in Table 1 below:

**Table 1**

| Strain name | Corresponding experimental example | Gene knockout | | | | Gene insertion |
|---|---|---|---|---|---|---|
| | | *skfA* | *sdpC* | *acdA* | *ackA* | *BCoAT* |
| BsS*-ΔskfA* | Comparative example 1 | - | + | + | + | - |
| BsS*-ΔsdpC* | Comparative example 2 | + | - | + | + | - |
| BsS-1 | Comparative example 3 | - | - | + | + | - |
| BsS-2 | Comparative example 4 | - | - | - | + | - |
| BsS-3 | Comparative example 5 | - | - | + | - | - |
| BsS-4 | Comparative example 6 | - | - | - | - | - |
| BsS-5 | Example 1 | - | - | - | - | + |
| BsS-6 | Example 2 | - | - | - | - | + |
| BsS-7 | Example 3 | - | - | - | - | + |

The strains provided by the examples and the comparative examples were subjected to performance comparison by specific tests below:

### (1) Comparison of growth rates of strains

Compared with the strain BsS, strain BsS*-ΔskfA,* strain BsS*-ΔsdpC* and strain BsS-1 had increased growth rates. After the strain BsS was cultured for 16 h, OD₆₀₀ tended to be stable. The strain BsS*-ΔskfA,* strain BsS*-ΔsdpC* and strain BsS-1 had similar growth rates and may tend to be stable after being cultured for about 10 h. Meanwhile, the dry weight of the bacteria was increased, the dry weight of the bacterium BsS was the lowest, the dry weight of the bacterium strain BsS-1 was the highest, and the dry weight of the bacterium strain BsS*-ΔskfA* took second place. In combination with Fig. 5, it can be known that by knocking out the genes *skfA* and *sdpC,* not only may the biomass and growth rates of the strains be increased, but also the yield of butyric acid may be improved, wherein the butyric acid yield of the strain BsS-1 was remarkably improved.

### (2) Testing of yields of acetic acid and butyric acid.

With the engineering bacterium strain BsS-1 as a chassis cell, gene *acdA* and gene *ackA* were further knocked out to obtain the engineering bacterium strain BsS-2 (with *acdA* knocked out), the engineering bacterium strain BsS-3 (with *ackA* knocked out) and the engineering bacterium strain BsS-4 (with *acdA* and *ackA* knocked out simultaneously) in sequence.

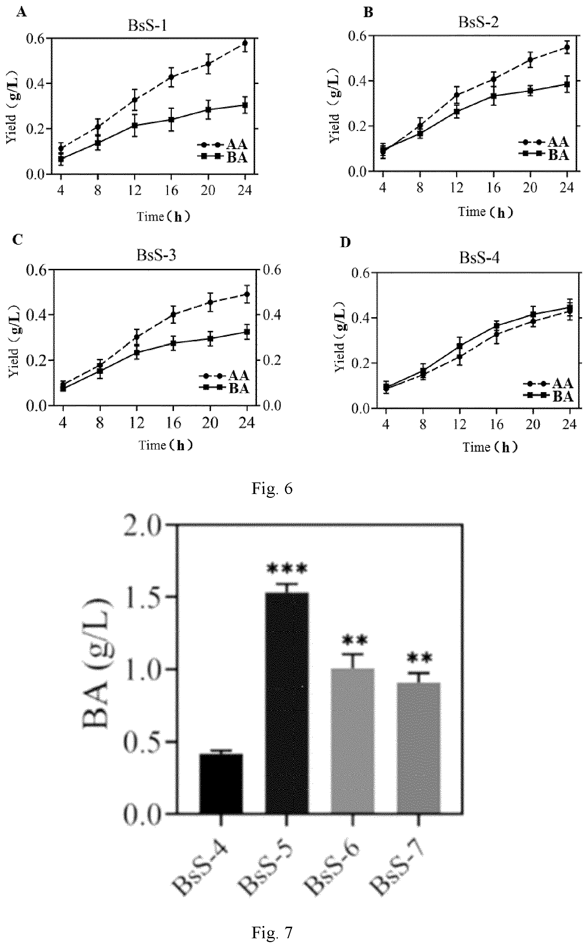

The butyric acid yield and the acetic acid yield of the strain BsS-1, strain BsS-2, strain BsS-3 and strain BsS-4 are shown in Fig. 6, wherein the graph A shows the curves corresponding to strain BsS-1, the graph B shows the curves corresponding to the strain BsS-2, the graph C shows the curves corresponding to the strain BsS-3, and the graph D shows the curves corresponding to the strain BsS-4. It can be known from the figures that the AA yield of the strain BsS-4 was remarkably reduced.

With the engineering bacterium strain BsS-4 as a chassis cell, gene *BCoAT* was inserted into different positions respectively to obtain the strain BsS-5, strain BsS-6 and strain BsS-7. The yield of the resultant butyric acid is shown in Fig. 7 from which it can be seen that after gene *BCoAT* was inserted, the butyric acid yields of the strain BsS-5, strain BsS-6 and strain BsS-7 were remarkably increased, the butyric acid yield of the strain BsS-5 was the highest, i.e., about 1.53 g/L, and the butyric acid yields of the strain BsS-6 and strain BsS-7 were 1.009 g/L and 0.91 g/L respectively.

The applicant declares that the above examples are intended to contribute to a better understanding of the present invention. However, the invention is not limited by the examples. It should be clear for those skilled in the art that any modifications or replacements that may be easily thought of by those skilled in the art within the technical scope disclosed by the present disclosure all fall in the protection scope and disclosure scope of the present disclosure.

## Claims

1. A butyric acid-producing *Bacillus subtilis* (*B. subtilis)* strain, wherein autolysis-related genes and acetic acid metabolism-related genes are deleted in the genome of the strain; and the genome further contains a butyric acid synthesis-related gene *BCoAT.*

2. The strain according to claim 1, wherein the genome further comprises a thiolase gene, a β-hydroxybutyryl-CoA dehydrogenase gene, a butyryl-CoA dehydrogenase gene and a crotonase gene; preferably, the thiolase gene, the β-hydroxybutyryl-CoA dehydrogenase gene, the butyryl-CoA dehydrogenase gene, the crotonase gene and the gene *BCoAT* on the genome are sequentially connected.

3. The strain according to claim 1 or 2, wherein the autolysis-related genes comprise any one or a combination of at least two of *skfA, sdpC, lytC* or *xpf,* preferably a combination of *skfA* and *sdpC*; preferably, the acetic acid metabolism-related genes comprise gene *acdA* and/or gene *ackA.*

4. A method for constructing the strain of any one of claims 1-3 comprising the following steps: using a two-plasmids CRISPR/Cas9 gene editing system to perform gene editing on a chassis cell, knocking out autolysis-related genes and acetic acid metabolism-related genes, and inserting a butyric acid synthesis-related gene *BCoAT* to obtain the strain.

5. The method according to claim 4, wherein the genome of the chassis cell comprises a thiolase gene, a β-hydroxybutyryl-CoA dehydrogenase gene, a butyryl-CoA dehydrogenase gene and a crotonase gene, which are sequentially connected; preferably, the chassis cell is *B. subtilis* SCK6.

6. The method according to claim 4 or 5, wherein in the two-plasmids CRISPR/Cas9 gene editing system, plasmids used comprise a pCas plasmid and a pTargetF plasmid; preferably, the selection marker on the pCas plasmid comprises a kanamycin marker gene; preferably, the pCas plasmid comprises a temperature-sensitive replicon region *rep*101*.*

7. The method according to claim 6, wherein the pTargetF plasmid comprises a promoter, a selection marker and sgRNA; preferably, the promoter comprises a P43 promoter; preferably, the selection marker comprises a spectinomycin marker gene.

8. The method according to claim 7, wherein the gene sequence of the sgRNA is shown as SEQ ID NO.1; preferably, the pTargetF plasmid further comprises homologous arms and a butyric acid synthesis-related gene *BCoAT*; preferably, the nucleotide sequence of the butyric acid synthesis-related gene *BCoAT* is shown as SEQ ID NO.2.

9. The method of any one of claims 4-8 comprising:
(1) constructing a two-plasmids CRISPR/Cas9 gene knockout editing system, wherein the gene editing system comprises a pCas plasmid containing a kanamycin marker gene and a pTargetF plasmid containing a spectinomycin marker gene;
(2) transforming the gene editing system in step (1) into a chassis cell to knock out autolysis-related gene *skfA* from the genome of the chassis cell, and eliminating the pTargetF plasmid after verifying the success of gene editing, wherein the genome of the chassis cell comprises a thiolase gene, a β-hydroxybutyryl-CoA dehydrogenase gene, a butyryl-CoA dehydrogenase gene and a crotonase gene;
(3) respectively knocking out the autolysis-related gene *sdpC,* acetic acid metabolism-related gene *acdA* and acetic acid metabolism-related gene *ackA,* and eliminating the two-plasmids CRISPR/Cas9 gene editing system for knockout after verifying the success of gene editing;
(4) constructing a two-plasmids CRISPR/Cas9 gene editing system for gene insertion, wherein the gene editing system comprises a pCas plasmid and a pTargetF plasmid and, the pTargetF plasmid contains a P43 promoter, a spectinomycin marker gene, sgRNA, homologous arms and a butyric acid synthesis-related gene *BCoAT* and, the gene sequence of the sgRNA is shown as SEQ ID NO.1 and, the nucleotide sequence of the butyric acid synthesis-related gene *BCoAT* is shown as SEQ ID NO.2; and
(5) transforming the gene editing system in step (4) into the chassis cell of which the gene *skfA,* the gene *sdpC,* the gene *acdA* and the gene *ackA* knocked out, and inserting the butyric acid synthesis-related gene *BCoAT* into the downstream of the crotonase gene to obtain the butyric acid-producing *B. subtilis* strain.

10. Application of the strain of any one of claims 1-3 in the preparation of butyric acid.
